# EUROPEAN PATENT APPLICATION

(11) **EP 4 491 114 A1**
(43) Date of publication of application: **15.01.2025**
(21) Application number: 23766682.1
(22) Date of filing: 01.03.2023
(51) Int. Cl.: A61B 5/1455, H01L 31/12, H01L 33/00

(54) **PROBE FOR PULSE OXYMETERS**

(30) Priority: 11.03.2022 JP 2022038273
(71) Applicant: STANLEY ELECTRIC CO., LTD., Tokyo 153-8636 (JP)
(72) Inventor: MIYAKE, Yasuyuki, Tokyo 153-8636 (JP)
(74) Representative: Pritzlaff, Stefanie Lydia
(86) International application number: PCT/JP2023/007656
(87) International publication number: WO 2023/171506

(57) **Abstract**

A probe for pulse oximeters is provided in which a light-emitting unit and a light-receiving unit do not protrude and a rise of a temperature of the light-emitting unit can be inhibited and it is not necessary to align the light-emitting unit and the light-receiving unit during mounting on a subject. A semiconductor light-emitting element of a bare chip is mounted on a ring-shaped flexible substrate. A semiconductor light-receiving element is mounted on a wiring on an outer circumferential surface of the substrate. The semiconductor light-receiving element receives light emitted from the semiconductor light-emitting element and passing through the subject and the transparent substrate.

## Description

### Technical Field

The present invention relates to a probe including a light source unit and a light-receiving unit for pulse oxymeters.

### Background Art

In pulse oxymeters, probes are worn on fingertips or the like, light (red light or infrared light) is emitted from light-emitting units in the probes to the fingertips or the like, and the light transmitted through the fingertips or the like is received by light-receiving units in the probes. Accordingly, in hemoglobin of blood, an abundance ratio of oxidized hemoglobin combined with oxygen to reduced hemoglobin not combined with oxygen is measured using a difference between absorption factors of the red light and the infrared light.

In medical sites, to prevent infection, disposable probes are used in many cases. In the disposable probes, light-emitting units and light-receiving units are disposed in adhesive tapes that have sizes covering fingers, and tapes are adhered and fixed to fingertips for use. In commercially available probes for pulse oxymeters, units on which LEDs packaged in advance on circuit substrates with rigidity such as glass epoxy are mounted are used as light-emitting units. Photodiodes are used in the light-receiving units. These photodiodes are mounted on adhesive tapes.

PTL 1 proposes a probe in which heat generated by an LED is trapped in an adhesive tape, a temperature of the probe-mounting unit of a subject is raised by 5 to 6°C, a thermally conductive and flexible holding material (adhesive tape) is used in order to solve a problem that there is concern of causing low-temperature burns to the subject.

### Citation List

### Patent Literature

PTL 1: JP3156114B

### Summary of Invention

### Technical Problem

The probe for a pulse oximeter disclosed in PTL 1 aims to inhibit heat of the light-emitting unit from being accumulated by using the thermally conductive holding material (adhesive tape) and to address a problem that the light-emitting unit protrudes from the adhesive tape by flexibility of the holding material. However, the size of the light-emitting unit on which the LED packaged in advance on a circuit substrate with rigidity such as glass epoxy is mounted protrudes at a height of 1.5 to 3 mm relative to the adhesive tape, and thus it is difficult to completely absorb the protrusion of the light-emitting unit with the flexibility of the adhesive tape.

When the protruding light-emitting unit and light-receiving unit are pressed against a finger by the adhesive tape, a blood flow delays, which makes it difficult for the heat from the light-emitting unit to escape to surroundings by the blood flow, and thus a temperature of the finger to rise. Due to an interaction between finger compression and temperature rise, it is more likely to leave a mark on the skin of the finger or cause irritation, and there is concern of developing low-temperature burns if worn for a long time. Medical workers need to exchange a probe frequently at about every 8 hours for adults and at intervals shorter than every 8 hours for newborns, elderly patients, or patients with delicate skin, to avoid low-temperature burns, which adds burden on the medical workers.

Therefore, it is important to provide a structure in which the light-emitting unit and the light-receiving unit do not protrude in order to prevent blood flow delay and to prevent low-temperature burns.

In the probe for the pulse oximeter in the related art, it is necessary to align optical axes of the light-emitting unit and the light-receiving unit accurately across a finger and attach an adhesive tape to the finger. Therefore, the alignment work during exchange of the probe burdens medical workers.

An object of the present invention is to provide a probe for pulse oximeters in which a light-emitting unit and a light-receiving unit do not protrude, a rise of a temperature of the light-emitting unit can be inhibited and it is not necessary to align the light-emitting unit and the light-receiving unit during mounting on a subject.

### Solution to Problem

To achieve the object, according to an aspect of the present invention, a probe for pulse oxymeters includes a flexible substrate, a semiconductor light-emitting element and a semiconductor light-receiving element mounted on the substrate, and a sealing material configured to seal the semiconductor light-emitting element and the semiconductor light-receiving element. A wiring is provided on a surface of the substrate. The semiconductor light-emitting element is a bare chip. The bare chip includes a semiconductor light-emitting layer and a pair of electrode layers for supplying a current to the semiconductor light-emitting layer. The bare chip is directly bonded on the wiring of the substrate by a bonding material. The semiconductor light-receiving element is mounted on a surface opposite to a surface facing a subject between both surfaces of the substrate and is bonded to the writing provided on the opposite surface. In the substrate, a region where at least the semiconductor light-receiving element is disposed is transparent. The semiconductor light-receiving element receives light emitted from the semiconductor light-emitting element and passing through the substrate.

### Advantageous Effects of Invention

In the probe for the pulse oximeters according to the present invention, the light-emitting unit and the light-receiving unit do not protrude from a substrate and the light-emitting unit is a bare chip. Therefore, thermal conductivity is good and a rise in temperature can be inhibited, and thus it is not necessary to align optical axes of the light-emitting unit and the light-receiving unit during mounting on a subject.

### Brief Description of Drawings

[FIG. 1] FIGS. 1(a) and 1(b) are sectional views illustrating a probe for pulse oxymeters according to a first embodiment of the present invention.
[FIG. 2] FIGS. 2(a) and 2(b) are perspective and side views illustrating the probe for the pulse oxymeters according to the first embodiment, respectively.
[FIG. 3] FIGS. 3(a) to 3(d) are diagrams illustrating processes of manufacturing the probe for the pulse oxymeters in Fig. 1(a) according to the first embodiment.
[FIG. 4] FIGS. 4(a) to 4(f) are diagrams illustrating processes of manufacturing the probe for the pulse oxymeters in Fig. 1(a) according to the first embodiment.
[FIG. 5] FIGS. 5(a) to 5(e) are diagrams illustrating processes of manufacturing the probe for the pulse oxymeters in FIG. 1(a) and FIG. 5(f) is a sectional view illustrating the manufactured probe according to the first embodiment.
[FIG. 6] FIG. 6 is a diagram illustrating a process of manufacturing the probe for the pulse oxymeters in Fig. 1(b) according to the first embodiment.
[FIG. 7] FIGS. 7(a-1) and 7(a-2) are top and sectional views illustrating a wiring 11b on which LED elements 21a and 21b of the probe for the pulse oxymeters are mounted according to the first embodiment, respectively, and FIGS. 7(b-1) and 7(b-2) are top and sectional views illustrating the wiring 11b on which the LED elements 21a and 21b of a probe for pulse oxymeters according to a second embodiment, respectively.
[FIG. 8] FIG. 8 is a side view illustrating a probe for pulse oxymeters according to a third embodiment.
[FIG. 9] FIG. 9 is a sectional view illustrating a probe for pulse oxymeters according to a fourth embodiment.
[FIG. 10] FIG. 10 is a table illustrating an experiment result when the probes for the pulse oxymeters according to the first and second embodiments and a comparative example.

### Description of Embodiments

Embodiments of the present invention will be described with reference to the drawings.

### <<<First Embodiment>>

### <<Configuration>>

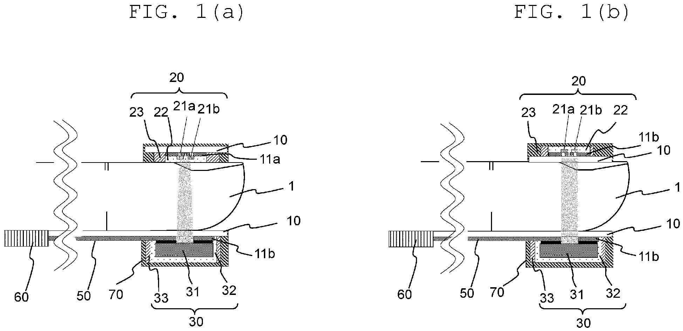

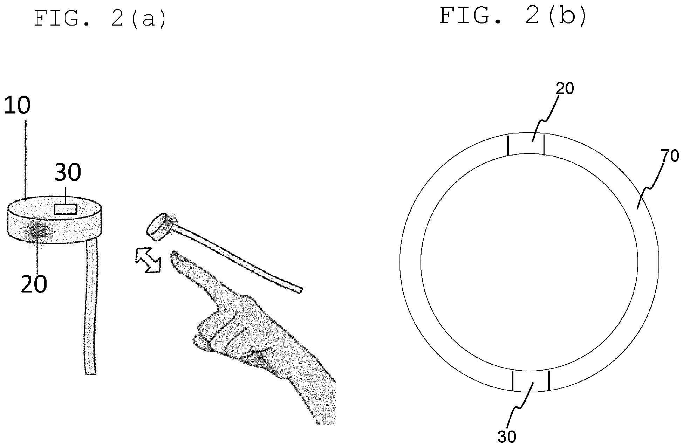

FIGS. 1(a) and 1(b) are sectional views illustrating planes including a central axis of a probe for pulse oxymeters according to the present embodiment. FIG. 2(b) is a side view.

As illustrated in FIGS. 1(a) and 1(b), the probe for the pulse oxymeters according to the present embodiment has a structure in which a wiring 11a or 11b is provided on a ring-shaped flexible substrate 10 and semiconductor light-emitting elements (LED elements) 21a and 21b of a bare chip are directly bonded on the wiring 11a or 11b of the substrate 10 by a bonding material. A surface on which the LED elements 21a and 21b are mounted may be an internal circumferential surface of the substrate 10 coming into contact with a subject 1 (FIG. 1(a)) or may be an outer circumferential surface of the substrate (FIG. 1(b)).

As the bonding material by which the LED elements 21a and 21b are bonded on the wiring 11a or 11b of the substrate 10, it is preferable to use a sintered body of metal particle. By using the sintered body of metal particles, it is possible to perform sintering at a temperature at which the flexible substrate 10 is not damaged.

A light-emitting wavelength of the LED element 21a is a wavelength of red light and a light-emitting wavelength of the LED element 21b is a wavelength of infrared light.

The circumferences of the LED elements 21a and 21b are sealed by a transparent sealing material 22.

Since a height (thickness) of the bare chip of the LED elements 21a and 21b can be generally set to 100 um or less, the bare chip can be buried with the transparent sealing material 22, and thus the surface of the sealing material 22 can be flattened. Accordingly, it is possible to configure a light-emitting unit 20 that does not have a protrusion pressing the skin of the subject 1.

The LED elements 21a and 21b of the bare chip can directly thermally conduct generated heat to the wiring 11a or 11b and can dissipate heat while conducting the heat to the wiring 11a or 11b. That is, the LED elements 21a and 21b of the bare chip have superior heat-drawing characteristics compared to a packaged LED since a member that becomes a bottle neck of heat conductivity or a member that can accumulate heat, such as a package substrate or a cavity, is not provided between the wiring 11a or 11b and the LED elements, and thus it is possible to efficiently dissipate the heat from the wiring 11a or 11b. Accordingly, it is possible to inhibit a rise in temperature of the light-emitting unit 20.

On the other hand, a semiconductor light-receiving element 31 provided in a light-receiving unit 30 is mounted on a surface (outer circumferential surface) opposite to a surface (inner circumferential surface) facing the subject 1 between both surfaces of the ring-shaped substrate 10 and is bonded to the wiring 11b provided on the outer circumferential surface. The circumferences of the semiconductor light-receiving element 31 are sealed by a transparent sealing material 32. In the substrate 10, a region where at least the semiconductor light-receiving element 31 is disposed is transparent. The semiconductor light-receiving element 31 receives light that is emitted from the LED elements 21a and 21b, passes through the subject 1, and further passes through the transparent substrate 10 and the sealing material 32.

The semiconductor light-receiving element 31 is mounted on the outer circumferential surface of the ring-shaped flexible substrate 10, so that the semiconductor light-receiving element 31 does not protrude to the inner circumferential surface of the substrate 10 and does not press the subject 1 even when the packaged semiconductor light-receiving element 31 is used. The bare chip that is not packaged as the semiconductor light-receiving element 31 can also be used.

Since the substrate 10 is in the ring form, the LED elements 21a and 21b and the semiconductor light-receiving element 31 can be mounted at positions facing across a space where the subject 1 inside the ring-shaped substrate 10 is disposed. Accordingly, when the LED elements 21a and 21b and the semiconductor light-receiving element 31 are mounted on the substrate 10, alignment for matching the optical axes can be completed. Therefore, when the probe according to the present embodiment is mounted on the subject 1, the ring-shaped substrate 10 may be fitted on a finger of the subject 1 and it is not necessary to align the LED elements 21a and 21b and the semiconductor light-receiving element 31. Accordingly, it is possible to reduce a burden of the alignment on the subject or a medical worker.

As described above, the LED elements 21a and 21b may be bonded on the wiring 11a on the internal circumferential surface of the substrate 10, as illustrated in FIG. 1(a), so that light may be emitted toward the subject 1 in a space in the ring-shaped substrate 10. The LED elements 21a and 21b may be bonded on the wiring 11b on the outer circumferential surface of the substrate 10 as illustrated in FIG. 1(b), so that light may be emitted toward the substrate 10 from the LED elements 21a and 21b and the emitted light may pass through the substrate 10 and be emitted toward the subject 1 in the space in the ring-shaped substrate 10. In the case of the configuration of FIG. 1(b), the substrate 10 in which a region where at least the LED elements 21a and 21b are mounted is transparent is used.

Note that, in the transparent sealing material 22 that seals the circumferences of the LED elements 21a and 21b and the transparent sealing material 32 that seals the circumference of the semiconductor light-receiving element, it is preferable that surfaces (side surfaces and outer circumferential surfaces) except for surfaces facing the subject 1 are covered with light-reflecting sealing materials 23 and 33. Accordingly, since light coming from the LED elements 21a and 21b can be reflected from the light-reflecting sealing material 23 and can travel toward the subject 1, an amount of light emitted to the subject 1 can be increased. Of the light passing through the subject 1 and arriving at the light-receiving unit 30, light not directly incident on a light-receiving surface of the semiconductor light-receiving element 31 can be reflected from the light-reflecting sealing material 33 and can be incident on the light-receiving surface. Therefore, it is possible to improve light reception efficiency.

As FIG. 2(b) illustrates a side view illustrating the probe according to the present embodiment, a protective sealing material 70 preferably seals a region on the substrate 10 other than the regions where the light-emitting unit 20 and the light-receiving unit 30 are disposed. The protective sealing material 70 covers and protects the substrate 10 and the wirings 11a and 11b on the substrate 10. The protective sealing material 70 preferably has a light-shielding property. By using a light-shielding protective sealing material, light leaking from a side surface of the light-reflecting sealing material 33 of the light-emitting unit 20 is prevented from propagating through the protective sealing material 70 and arriving at the light-receiving unit 30.

The transparent sealing materials 22 and 32, the light-reflecting sealing materials 23 and 33, and the protective sealing material 70 are preferably elastic materials. The light-reflecting sealing materials 23 and 33 and the protective sealing material 70 can each be the same material that has a light-reflecting property and a light-shielding property.

In this way, in the probe for the pulse oxymeters according to the present embodiment, the LED elements 21a and 21b of the bare chip are mounted as the light-emitting unit 20 and the semiconductor light-receiving element 31 is mounted as the light-receiving unit 30 directly on the ring-shaped flexible substrate 10 in which wirings are provided. In this way, since the LED elements 21a and 21b of the light-emitting unit 20 and the semiconductor light-receiving element 31 of the light-receiving unit 30 do not directly come into contact with a finger of the subject 1, unevenness of a portion coming into contact with the finger during measurement can be reduced, and thus does not press the finger. In particular, in the structure of FIG. 1(b) in which the LED elements 21a and 21b and the semiconductor light-receiving element 31 are mounted on the outer circumferential surface of the ring-shaped substrate 10, the light-emitting unit 20 and the light-receiving unit 30 are disposed together on an opposite side to the finger during mounting on the finger. Therefore, it is possible to obtain the effect that the unevenness coming into contact with the finger is not formed and does not press the finger.

In the ring-shaped probe according to the present embodiment, a power supply and signal acquisition wiring 50 that supplies power to the LED elements 21a and 21b and extracts an output signal of the semiconductor light-receiving element 31 is connected to the wirings 11a and 11b and a connection terminal 60 is mounted on a tip end of the wiring 50.

When the probe is used, as illustrated in FIG. 2(a), the probe is fitted in the finger of the subject 1, the terminal 60 is connected to a pulse oxymeter device, and power is supplied to the LED elements 21a and 21b via the power supply and signal acquisition wiring 50 and the wiring 11a or 11b. Red light and infrared light output from the LED elements 21a and 21b pass through the finger and are received by the semiconductor light-receiving element 31. An output of the semiconductor light-receiving element 31 is input to the pulse oxymeter via the wiring 11b and the power supply and signal acquisition wiring 50. The pulse oxymeter calculates and displays a ratio (SpO₂) of oxidized hemoglobin in the hemoglobin of blood from an intensity ratios or the like of the red light and the infrared light from an output of the semiconductor light-receiving element 31.

### <<Detailed Structure and Manufacturing Method>>

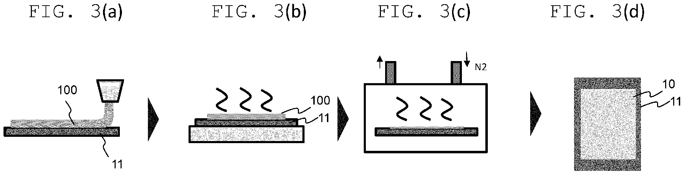

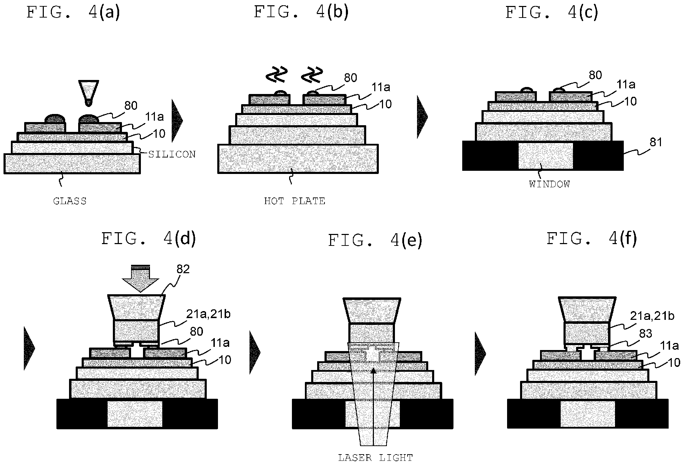

A detailed structure and a manufacturing method for the probe for the pulse oxymeters according to the present embodiment will be described with reference to FIGS. 3 and 4. First, a manufacturing method for the probe that has the structure of FIG. 1(a) will be described.

### (Process of Manufacturing Substrate 10 with Wirings 11a and 11b)

As illustrated in FIG. 3(a), an uncured transparent polyimide layer 100 with a constant thickness (for example, 70 µm) is applied on a copper foil 11 (for example, a thickness of 12 µm) using a die coater.

As illustrated in FIG. 3(b), the copper foil 11 with the uncured polyimide layer 100 is heated at a predetermined temperature and for a predetermined time (for example, 100°C and 10 min) using a hot plate or the like and temporary drying is performed. At this time, a hole may be formed in the hot plate and the copper foil 11 with the polyimide layer 100 may be heated while being adsorbed to the hot plate. A frame may be placed on the top of the copper foil 11 with the polyimide layer 100 and may be pressed down as needed.

As illustrated in FIG. 3(c), the copper foil 11 with the polyimide layer 100 is heated at a predetermined temperature and for a predetermined time (for example, 260°C and 60 min) using an oven under a nitrogen atmosphere and main drying is performed. Accordingly, the transparent substrate 10 (transparent polyimide with a thickness of 35 µm) integrated with the copper foil 11 is formed (FIG. 3(d)).

Subsequently, the copper foil on the substrate 10 in FIG. 3(d) is processed by etching to form a pattern of the wiring 11a on which the LED elements 21a and 21b are mounted, a pattern of the wiring 11b on which the semiconductor light-receiving element 31 is mounted, and a pattern of the power supply and signal acquisition wiring 50. A gold plate layer is formed on the surface of the processed copper foil by electroless plating. Here, both the wirings 11a and 11b are formed by processing the copper foil of the same surface on the substrate 10.

### (Process of Mounting LED Elements 21a and 21b)

The LED elements 21a and 21b are mounted on the wiring 11a on the substrate 10. The LED elements 21a and 21b are of a flip-chip type in which electrodes are on the same side and light is emitted from an opposite side to the electrodes.

As illustrated in FIG. 4(a), an Au ink 80 in which gold nanoparticles with an average particle diameter of 30 nm are dispersed in a solvent (glycerin) at a concentration of 82 wt% is prepared and is applied to portions on which the LED elements 21a and 21b of the wiring 11a are mounted using a dispenser. At this time, it is preferable to mount the substrate 10 on a silicon substrate and additionally on a glass substrate.

Subsequently, As illustrated in FIG. 4(b), heating is performed at a predetermined temperature and for a predetermined time (for example, 50°C and 45 to 60 min) on the hot plate and the Au ink 80 is dried.

As illustrated in FIG. 4(c), the substrate 10 is set in an optical bonding device 81. As illustrated in FIG. 4(d), the electrodes of the LED elements 21a and 21b are mounted on the Au ink 80, and the LED elements 21a and 21b are pressed against the wiring 11a at a predetermined pressure using a load collet 82.

As illustrated in FIG. 4(e), blue laser light with a predetermined beam diameter is emitted from a lower surface of the substrate 10 to the region where the Au ink 80 of the wiring 11a is mounted. In the wiring 11a, the region irradiated with the blue laser light absorbs the blue laser light to be heated and the heat is conducted to the Au ink 80 to heat the Au ink 80. Since an average particle diameter of 30 nm of the gold particles in the Au ink 80 is small, the gold particles are sintered into a sintered body at a temperature lower than a melting point of Au, and thus a bonding material 83 that bonds the wiring 11a to the electrodes of the LED elements 21a and 21b is formed.

At this time, since the substrate 10 is transparent, the substate 10 does not absorb the blue laser light and is not directly heated. The heat of the wiring 11a heated by the blue laser light is thermally conducted to a region of the wiring 11a where the blue laser light is not emitted, and thus the heat rapidly dissipates. Accordingly, the LED elements 21a and 21b can be mounted on the wiring 11a with the bonding material 83 of the sintered body of the gold particles by optical bonding without damage to the LED elements 21a and 21b and the substrate 10 made of a resin.

### (Process of Mounting Semiconductor Light-Receiving Element 31)

The semiconductor light-receiving element 31 is a flip-chip type photodiode in which a pair of electrodes are on the same surface side, and a photodiode in which there is a light-receiving surface between a pair of electrodes is used.

The semiconductor light-receiving element 31 is mounted on the wiring 11b of the substrate 10. As a mounting method, the semiconductor light-receiving element 31 may be mounted by the optical bonding method as illustrated in FIGS. 4(a) to 4(f). Since the semiconductor light-receiving element 31 has a larger size than the LED elements 21a and 21b, the bonding may be performed by soldering.

### (Sealing and Assembling Processes)

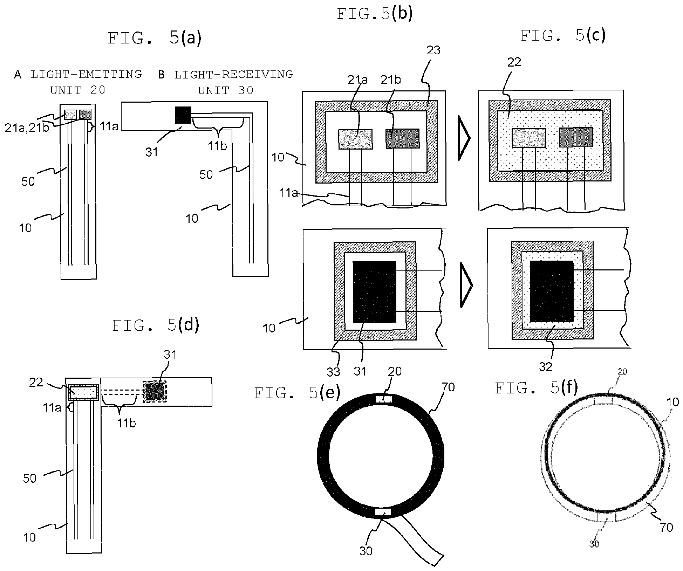

As illustrated in FIG. 5(a), the substrate 10 in which the wiring 11a on which the LED elements 21a and 21b are mounted and the power supply and signal acquisition wiring 50 connected to the wiring 11a are formed and the substrate 10 in which the wiring 11b on which the semiconductor light-receiving element 31 is mounted and the power supply and signal acquisition wiring 50 connected to the wiring 11b are formed are cut out as different parts A and B.

As illustrated in FIG. 5(b), an uncured white resin in which titanium oxide, zinc oxide, or aluminum oxide is dispersed in a silicone resin is prepared and is applied at a position away from the LED elements 21a and 21b and the semiconductor light-receiving element 31 by a predetermined distance in a frame form surrounding each of the LED elements 21a and 21b and the semiconductor light-receiving element 31 to form the uncured light-reflecting sealing materials 23 and 33. The height of the light-reflecting sealing materials 23 and 33 in the frame form becomes a predesigned height higher than the height of the LED elements 21a and 21b and the semiconductor light-receiving element 31.

As illustrated in FIG. 5 (c), a transparent silicon resin is injected in the formed light-reflecting sealing materials 23 and 33 in the frame form to form the uncured transparent sealing materials 22 and 32. Thereafter, the substrate 10 is heated at a predetermined temperature and for a predetermined time (for example, 150°C and 4 h) to cure the light-reflecting sealing materials 23 and 33 and the transparent sealing materials 22 and 32. Accordingly, here, the light-reflecting sealing materials 23 and 33 and the transparent sealing materials 22 and 32 with a thickness of 30 µm in an optical axis direction are formed.

As illustrated in FIG. 5(d), rear surfaces of the part A of the substrate 10 on which the LED elements 21a and 21b are mounted and the part B of the substrate 10 on which the semiconductor light-receiving element 31 is mounted are pasted. An adhesive layer of a resin or an adhesive tape can be used for the pasting. The adhesive layer of the pasting preferably has a light-shielding property.

The substrate 10 is set to be in a ring form according to a mold in which ring-shaped recesses are formed. At this time, the power supply and signal acquisition wiring 50 and the portion of the substrate 10 on which the power supply and signal acquisition wiring 50 is mounted are set to be pulled out from the mold. The circumference of the substrate 10 is filled with a light-shielding silicon resin. Accordingly, the light-shielding protective sealing material 70 is formed. At this time, a mold is formed on the inner circumferential surface of the light-emitting unit 20 and the light-receiving unit 30 so that the light-shielding protective sealing material 70 is not attached.

The protective sealing material 70 in the mold is cured while being pressurized at a predetermined temperature and for a predetermined time (for example, 150°C and 4 h). After the curing, as illustrated in FIG. 5(e), the entire ring-shaped substrate 10 covered with the protective sealing material 70 is extracted from the mold. FIG. 5(f) is a sectional view illustrating the ring-shaped substrate 10 covered with the protective sealing material 70 in FIG. 5 (e) . As illustrated in FIG. 5(f), the protective sealing material 70 covers an inner circumferential surface side of the substrate 10 in the circumference of the light-emitting unit 20, covers an outer circumferential surface side of the substrate 10 in the circumference of the light-receiving unit 30, and covers both the inner circumferential surface side and the outer circumferential surface side of the substrate 10 between the light-emitting unit 20 and the light-receiving unit 30, and thus the outer appearance of the protective sealing material 70 is formed in a smooth ring shape.

Finally, the connection terminal 60 is connected to a tip end of the power supply and signal acquisition wiring 50 pulled out from the ring-shaped substrate 10.

As described above, as illustrated in FIG. 5(e), the ring-shaped probe for the pulse oxymeters according to the present embodiment is completed.

Next, a manufacturing method for the probe that has the structure of FIG. 1(b) will be described.

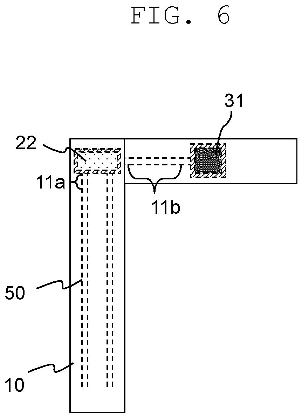

A difference between the probe that has the structure of FIG. 1(b) and the probe that has the structure of FIG. 1(a) is that both the LED elements 21a and 21b and the semiconductor light-receiving element 31 are mounted on the wiring 11b on the outer circumferential side of the substrate 10. Accordingly, in the manufacturing process for the probe that has the structure of FIG. 1(a), it is not necessary to cut out and divide the substrate 10 into the two parts A and B of the substrate in the process of FIG. 5(a) and it is not necessary to paste the two parts A and B of the substrate 10 in the process of FIG. 5(d). Accordingly, in the process of FIG. 5(a) of the manufacturing method for the probe that has the structure of FIG. 1(a), as illustrated in FIG. 6, one part in which both the LED elements 21a and 21b and the semiconductor light-receiving element 31 are mounted may be cut out. Note that, since the pattern of the wiring 11b is different from that of the probe that has the structure of FIG. 1(a), appropriate design is required.

The other processes are similar to those of the probe that has the structure of FIG. 1(a), and thus description thereof will be omitted. Note that, in the process of FIG. 5(e), the protective sealing material 70 is formed in a ring shape covering the outer circumferential surface of the substrate 10 in the circumference of the light-emitting unit 20, the circumference of the light-receiving unit 30, and a region between the light-emitting unit 20 and the light-receiving unit 30.

As described above, it is possible to manufacture the probe that has the structure of FIG. 1(b).

### <<<Second Embodiment>>>

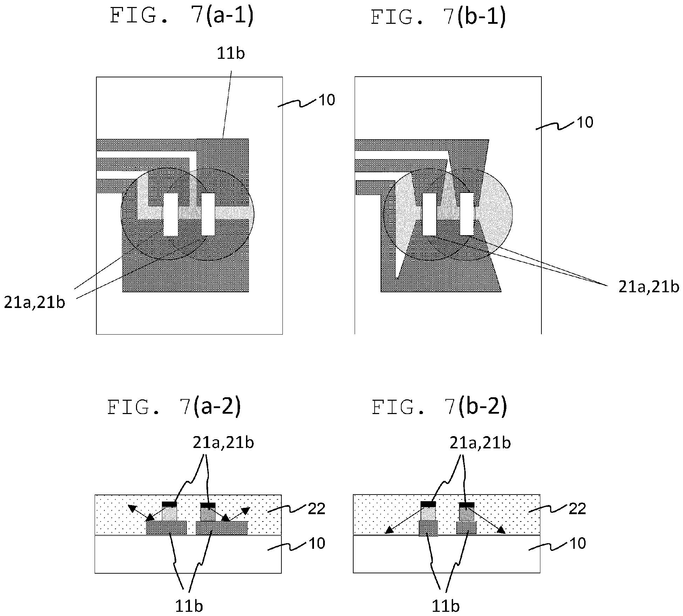

A probe for pulse oxymeters according to a second embodiment will be described with reference to FIG. 7.

In the probe that has the structure of FIG. 1(b) according to the first embodiment, the LED elements 21a and 21b of the light-emitting unit 20 are mounted on the wiring 11b of the outer circumferential surface of the ring-shaped substrate 10. Therefore, light emitted from the LED elements 21a and 21b passes through a gap of the wiring 11b and through the transparent substrate 10 and is emitted to the subject 1. In order to increase an amount of light emitted from the light-emitting unit 20 to the subject 1, it is preferable to decrease the width of the wiring 11b and increase the gap of the wiring 11b. On the other hand, the wiring 11b also has a role of diffusing and dissipating heat generated in the LED elements 21a and 21b. In order to prevent low-temperature burns or the like and to reduce a burden on a patient, an area of the wiring 11b of portions in which the LED elements 21a and 21b are mounted is preferably large.

Accordingly, according to the second embodiment, the wiring 11b to which the LED elements 21a and 21b are bonded has a shape in which a width increases away from the LED elements 21a and 21b.

This will be described with reference to FIG. 7. FIGS. 7(a-1) and 7(a-2) are top and sectional views illustrating a region where the LED elements 21a and 21b of the wiring 11b according to the first embodiment are mounted, respectively. FIGS. 7(b-1) and 7(b-2) are top and sectional views illustrating a region where the LED elements 21a and 21b of the wiring 11b according to the second embodiment are mounted, respectively.

The wiring 11b in FIG. 7(a-1) according to the first embodiment has a rectangular shape with a constant width. The wiring 11b in FIG. 7(b-1) according to the second embodiment has a trapezoidal shape with a narrowed width of the wiring 11b in a portion where the LED elements 21a and 21b are mounted and a width increasing away from the LED elements 21a and 21b.

Accordingly, an aera covered by the wiring 11b is small in a region on the substrate 10 to which the light of the LED elements 21a and 21b is emitted. Accordingly, as illustrated in FIG. 7(b-2), of the light emitted from the LED elements 21a and 21b, an amount of light reflected from the surface of the wiring 11b can be less than that of the rectangular wiring 11b in FIG. 7(b-1).

Accordingly, the wiring 11b according to the second embodiment can increase an amount of light incident on the substrate 10 from the gap of the wiring 11b, leaking from the substrate 10, and emitted to the subject 1.

Since the width of the wiring according to the second embodiment is widened away from the LED elements 21a and 21b, an operational effect of diffusing and dissipating the heat generated in the LED elements 21a and 21b can be maintained. Accordingly, it is possible to increase the amount of light toward the subject 1 while maintaining the heat dissipation characteristics.

FIG. 7(b-1) illustrates an example of the wiring 11b that has a trapezoidal shape with a continuously increasing width, but the wiring 11b may have a shape increasing in a step manner.

The other configuration and manufacturing method than the shape of the wiring 11b of the probe according to the second embodiment is similar to those of the first embodiment, and thus description thereof will be omitted.

### <<<Third Embodiment>>>

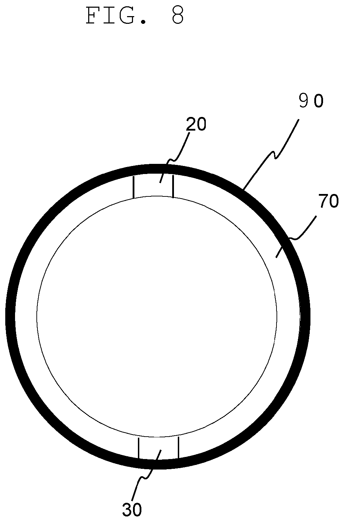

A probe for pulse oxymeters according to a third embodiment will be described with reference to FIG. 8.

In the probe according to the third embodiment, as illustrated in FIG. 8, a metal film 90 is disposed on outer circumferential surfaces of the light-emitting unit 20, the light-receiving unit 30, and the light-shielding protective sealing material 70.

The metal film 90 can enhance a heat dissipation operational effect of the probe since heat generated by the light-emitting unit 20 can be dissipated. The metal film 90 can also serve as an electromagnetic shield film, and thus electromagnetic noise from the outside can be prevented from being mixed in the light-emitting unit 20, the light-receiving unit 30, and the wirings 11a and 11b.

As a material of the metal film 90, a metal that has high conductivity, such as copper or aluminum can be used. A film thickness is preferably in the range of 1 µm to 100 µm.

The metal film 90 can be formed, after a process of forming the protective sealing material 70 in FIG. 5(e), by vapor deposition or a plating technique. A metal foil may be disposed and simultaneously cured along the outer circumference when the protective sealing material 70 is formed, or a metal ring separately generated in advance using a metalworking technique may be disposed on outside of the protective sealing material 70 to be fixed by a solder, an adhesive, or the like after the protective sealing material 70 in FIG. 5(e) is formed.

In the third embodiment, the structure in which the metal film 90 is disposed on the uppermost surface of the probe is illustrated. However, before the process of forming the protective sealing material 70 in FIG. 5(e), the metal film may be formed. Subsequently, the process in FIG. 5(e) may be performed and the protective sealing material 70 may be formed on the metal film 90.

### <<<Fourth Embodiment>>>

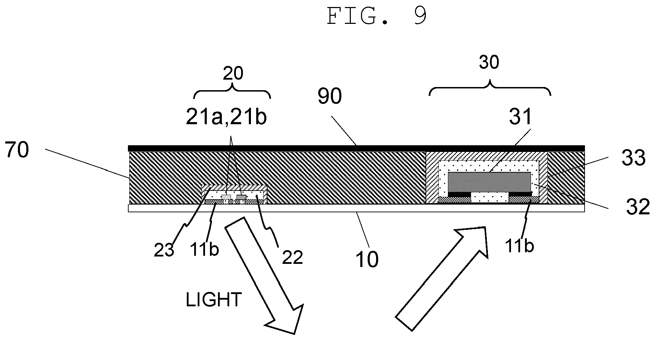

A probe for pulse oxymeters according to a fourth embodiment will be described with reference to FIG. 9.

In the first to third embodiments, the substrate 10 is the ring-shaped probe. In the fourth embodiment, a planar reflective probe to which the structure in FIG. 1(b) according to the first embodiment is applied will be described. The planar probe is attached to, for example, a sole of a newborn infant for use. Of light emitted from the light-emitting unit 20, light reflected from the subject 1 is received by the light-receiving unit 30.

The probe according to the fourth embodiment has a structure in which the ring-shaped substrate 10 having the structure of FIG. 1(b) is planar and has a structure in which the bare chip of the LED elements 21a and 21b and the semiconductor light-receiving element 31 are directly mounted on the wiring 11b of the upper surface of the substrate 10.

Accordingly, in the reflective probe, an unevenness is not provided on the surface coming into contact with the subject 1, and thus it is possible to obtain the effect similar to that of the first embodiment.

The other structure, the other operational effect, and the like except the planar shape and that the reflected light is received are similar to those of the first embodiment, and detailed description thereof will be omitted.

In the structure in FIG. 9, the metal film 90 according to the third embodiment is also disposed.

### <<<Verification of Effects of Probe According to Present Embodiment>>>

To evaluate a risk of low-temperature burns, an experiment was executed in which the probe according to the first embodiment (a cross-sectional structure in FIG. 1(a)), the probe according to the second embodiment (a cross-sectional structure in FIG. 1(b)) according to the second embodiment, and a commercially available probe (manufactured by As One Corporation, model number: NF503-16) according to a comparative example were mounted on a hand mannequin to measure a rise in temperature of a mounting unit.

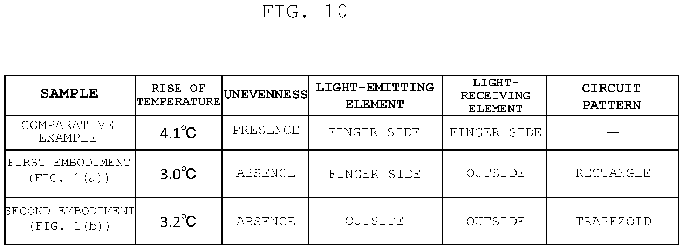

In each probe, LED elements of red light and infrared light were turned on simultaneously for continuous conduction at 5 mA and a rise of temperature of the mounting unit of the hand mannequin at that time was measured. A result is shown in the table of FIG. 10.

As apparent from FIG. 10, it was confirmed that the probes according to the first and second embodiments further inhibited the rise of temperature than the comparative example.

Since the rise of temperature is a cause of irritation or low-temperature burns in the subject 1, the probes according to the present embodiment can reduce a burden on the subject 1.

### <<<Application to Product>>>

The probe for pulse oxymeters according to the present embodiment can also be used as a light source unit of another wearable device such as a smart ring that optically acquires biological information.

### Reference Signs List

1: subject
10: substrate
11: copper foil
11a: wiring
11b: wiring
20: light-emitting unit
21a: LED element
21b: LED element
22: transparent sealing material
23: light-reflecting sealing material
30: light-receiving unit
31: semiconductor light-receiving element
32: transparent sealing material
33: light-reflecting sealing material
50: wiring
60: connection terminal
70: protective sealing material
80: ink
81: optical bonding device
82: load collet
83: bonding material
90: metal film
100: polyimide layer

## Claims

1. A probe for pulse oxymeters comprising:
a flexible substrate;
a semiconductor light-emitting element and a semiconductor light-receiving element mounted on the substrate; and
a sealing material configured to seal the semiconductor light-emitting element and the semiconductor light-receiving element,
wherein a wiring is provided on a surface of the substrate,
wherein the semiconductor light-emitting element is a bare chip including a semiconductor light-emitting layer and a pair of electrode layers for supplying a current to the semiconductor light-emitting layer, and the bare chip is directly bonded onto the wiring of the substrate by a bonding material,
wherein the semiconductor light-receiving element is mounted on a surface opposite to a surface facing a subject between both surfaces of the substrate and is bonded to the wiring provided on the opposite surface,
wherein, in the substrate, a region where at least the semiconductor light-receiving element is disposed is transparent, and
wherein the semiconductor light-receiving element receives light emitted from the semiconductor light-emitting element and passing through the transparent substrate.

2. The probe for the pulse oxymeters according to claim 1, wherein the substrate is in a ring shape.

3. The probe for the pulse oxymeters according to claim 2, wherein the semiconductor light-receiving element is mounted on an outer circumferential surface of the substrate at a position facing the semiconductor light-emitting element across a space in the ring-shaped substrate.

4. The probe for the pulse oxymeters according to claim 2, wherein the semiconductor light-emitting element is bonded onto the wiring on an inner circumferential surface of the substrate and emits light toward a space in the ring-shaped substrate.

5. The probe for the pulse oxymeters according to claim 1,
wherein, in the substrate, a region where the semiconductor light-emitting element is mounted is transparent, and
wherein the semiconductor light-emitting element is bonded onto the wiring on one surface of the substrate, the semiconductor light-emitting element emits light toward the substrate, and the emitted light is emitted toward the subject through the transparent substrate.

6. The probe for the pulse oxymeters according to claim 1, wherein the bonding material is a sintered body of metal particles.

7. The probe for the pulse oxymeters according to claim 1, wherein the sealing material includes a transparent sealing material that seals circumferences of the semiconductor light-emitting element and the semiconductor light-receiving element, a light-reflecting sealing material that covers a side surface of the transparent sealing material, and a light-shielding sealing material that seals a region of the substrate where the semiconductor light-emitting element and the semiconductor light-receiving element are not mounted.

8. The probe for the pulse oxymeters according to claim 7, wherein a metal film is disposed on an upper surface of the sealing material.

9. The probe for the pulse oxymeters according to claim 1, wherein the wiring to which the semiconductor light-emitting element is bonded has a width increasing away from the semiconductor light-emitting element.
